# EUROPEAN PATENT APPLICATION

(11) **EP 2 543 712 A1**
(43) Date of publication of application: **09.01.2013**
(21) Application number: 11750744.2
(22) Date of filing: 03.03.2011
(51) Int. Cl.: C09K 3/00, C07D 311/62, C07H 13/08, C09K 5/00

(54) **SUPERCOOLING PROMOTING AGENT**

(30) Priority: 04.03.2010 JP 2010048440
(71) Applicant: National University Corporation Hokkaido University, Sapporo-shi, Hokkaido 060-0808 (JP); Amino Up Chemical Co., Ltd., Hokkaido 004-0839 (JP)
(72) Inventor: FUJIKAWA Seizo, Hokkaido 060-0808 (JP); FUKUSHI Yukiharu, Hokkaido 060-0808 (JP); ARAKAWA Keita, Hokkaido 060-0808 (JP); NISHIOKA Hiroshi, Sapporo-shi Hokkaido 004-0839 (JP)
(74) Representative: Moreland, David
(86) International application number: PCT/JP2011/054892
(87) International publication number: WO 2011/108635

(57) **Abstract**

The present invention discloses a supercooling promoting agent comprising a tannin for producing practical water which does not freeze. As the tannin, a hydrolyzable tannin such as 2, 3, 6-tri-O-galloyl-α, β-D-hamamelose, 1, 2, 6-tri-O-galloyl-β-D-glucose, and a vitrification liquid, each of which contains the supercooling promoting agent are useful as a solution or the like for storig a biological material at low temperature,

## Description

### Technical Field

The present invention relates to a supercooling promoting agent. More specifically, the present invention relates to a supercooling promoting agent comprising a tannin having an activity to supercool water by inhibiting formation of ice nuclei at a low concentration, and to a nonfreezing liquid and a vitrification solution each containing the tannin.

### Background Art

A substance which inhibits freezing of water at below the freezing point stably for a long period of time is expected to be applied in various industrial fields. However, at present, a substance which, when added at a low concentration, supercools water by at least 10°C or more for 1 day or more to such an extent that the substance may be expected to be industrially applied is not known.

Cell water of a tree which grows in a cold region is known to maintain a liquid state at low temperature. In the tree, water in xylem parenchyma cells is supercooled to -40°C by physical properties of water due to water droplets separated from the outside world (Non-patent Document 1). Cell walls which surround the xylem parenchyma cells act as a barrier for preventing dehydration from the cells and invasion of extracellular ice into the cells. Therefore, even if ice is formed outside the cells, water in the cells is considered to behave as water droplets isolated from the outside world to undergo supercooling. Further, a phenol compound (flavonoid) contained in an overwintering plant is known to seem to have a supercooling activity and is suggested to act as a freezing protecting substance (Non-patent Document 2). There have been disclosed: use of the flavonoid in a frozen medium for culturing germ cells or the like (Patent Document 1); and use of the flavonoid as a component of an antifreeze solution used as a cooling solution for an internal-combustion engine or the like (Patent Document 2). It should be noted that many kinds of flavonoid glycosides are known to be present in plants including trees and biological substances as secondary metabolites (Non-patent Document 3).

The inventors of the present invention have started comprehensive studies to elucidate a mechanism for maintaining a liquid state of cell water in a tree which grows in a cold region at low temperature, and first have clarified that, with regard to supercooling ability which is stably shown by xylem parenchyma cells at -40°C over several weeks (the ability is referred to as deep supercooling ability in distinction from temporal supercooling), the ability to supercool water by 20°C is provided because of the fact that water is physically isolated, but the ability to supercool water by the residual 20°C is provided by a certain substance present in the cells (Non-patent Document 4).
Subsequently, an increase in the supercooling activity by a molar freezing-point depression has been determined from the malting temperature of the cells and the concentration of water in the cells. As a result, the inventors have concluded that the increase in the supercooling activity by an effect of the intracellular concentration is about 2 to 3°C, and the supercooling activity for the residual 17 to 18°C is caused not by the above-mentioned physical factors but by a certain substance present in the cells. The increase in the supercooling limit temperature of the xylem parenchyma cells was accompanied with characteristic gene expression and protein expression caused by the gene expression, but the protein was found to have no supercooling promoting activity. In addition, there was no carbohydrate which is characteristically accumulated only in the xylemparenchyma cells, and respective carbohydrates accumulated in the cells were found to have no supercooling promoting activity.

On the other hand, secondary metabolites other than proteins and carbohydrates extracted from the xylem parenchyma cells exhibited high supercooling activities, and the supercooling activities were detected in a crude extract extracted from the xylem parenchyma cells with ethanol and all fractions obtained by purifying the crude extract in several steps. As a result, xylem parenchyma cells capable of deep-supercooling were found to include various substances exhibiting high supercooling activities.
The inventors of the present inventionhave identified flavonol glycosides which exhibit supercooling activities in a range of 3 to 9°C from the fractions exhibiting supercooling activities, and applied for a patent previously (Patent Document 3).

### Citation List

### Patent Document

[Patent Document 1] JP 2000-500327 (WO 97/14785)
[Patent Document 2] WO 2004/074397 (US 2006/0038159)
[Patent Document 3] WO 2008/007684 (US 2009/0302265)

### Non-patent Document

[Non-patent Document 1] Chemistry and Biology, vol. 43, No. 5, 280-282 (2005)
[Non-patent Document 2] Chemistry and Biology, vol. 37, No. 12, 778-780 (1999)
[Non-patent Document 3] Flavonoids Chemistry, Biochemistry and Applications, CRC Press Taylor and Francis Group (2006)
[Non-patent Document 4] Role of intracellular contents to facilitate supercooling capability in beech (Fagus crenata) xylem parenchyma cells. CryoLetters, 27 (5), 305-310 (2006)

### Summary of Invention

### Problem to be Solved by the Invention

An object of the present invention is to provide a novel supercooling promoting agent for preparing practical unfrozen water by identifying a component (substance) which exhibits a high supercooling activity, other than the above-mentioned flavonoid glycoside, from a fraction which is extracted from xylem parenchyma cells and exhibits a supercooling activity.

### Means to Solve the Problem

The inventors of the present invention have tried identification of an effective active component from a fraction extracted based on a supercooling activity as an index from xylem parenchyma cells of *Cercidiphylium japonicum having xylem parenchyma* cells capable of deep-supercooling to -40°C. As a result, the inventors have confirmed that hydrolyzable tannins 2,3,6-tri-O-galloyl-α,β-D-hamamelose (kurigalin), 1,2,6-tri-O-galloyl-β-D-glucose, and 1,2,3,6-tetra-O-galloyl-β-D-glucose have activities to supercool water by 3.5 to 4.6°C. Based on the findings, the inventors have examined whether or not other hydrolyzable tannins and condensed tannins have supercooling activities. As a result, the inventors have confirmed that tannic acid derived from nutgall, which is a hydrolyzable tannin; condensed catechin-type tannins lychee fruit oligomerized polyphenol (Oligonol, trade name of a lychee fruit oligomerized polyphenol manufactured by Amino Up Chemical Co., Ltd.), lychee fruit polyphenol (LFP), and grape seed polyphenol (GSP); and catechins such as tea catechin, catechin (C), epicatechin (EC), epicatechin gallate (ECG), epigallocatechin (EGC), gallocatechin gallate (GCG), and epigallocatechin gallate (EGCG) have supercooling activities, thus completing the present invention.

That is, the present invention relates to a supercooling promoting agent comprising a tannin according to 1 to 8 below, a nonfreezing liquid according to 9 to 13 below, and a vitrification solution according to 14 to 16 below.
1. A supercooling promoting agent comprising a tannin.
2. The supercooling promoting agent according to 1 above, in which the tannin is a sugar ester-type hydrolyzable tannin having a partial structure in which one or more hydroxy groups of a sugar having 6 carbon atoms are each ester-bonded to a substituted benzoyl group selected from structures represented by the following formulae (1) to (6): 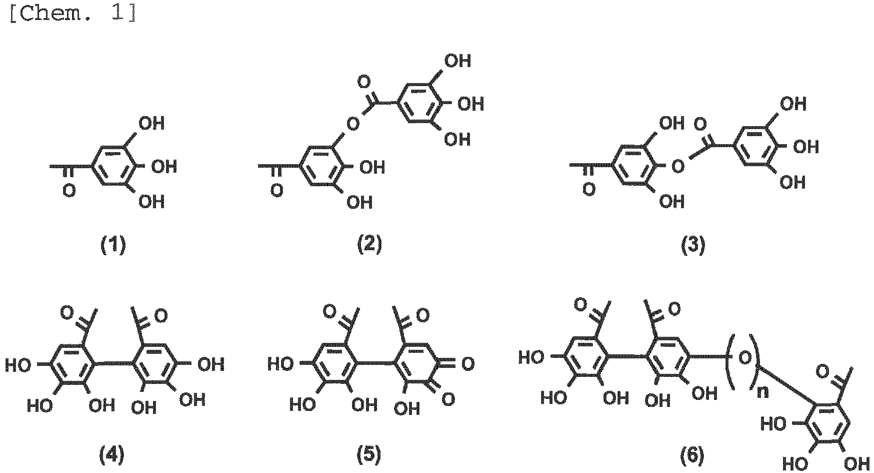 (wherein, n represents 0 or 1).
3. The supercooling promoting agent according to 2 above, in which the hydrolyzable tannin is 2,3,6-tri-O-galloyl-α,β-D-hamamelose, 1,2,6-tri-O-galloyl-β-D-glucose, or 1,2,3,6-tetra-O-galloyl-β-D-glucose.
4. The supercooling promoting agent according to 2 above, in which the hydrolyzable tannin is tannic acid derived from nutgall.
5. The supercooling promoting agent according to 1 above, in which the tannin is a catechin-type tannin containing, as a partial structure, a flavan-3-ol skeleton represented by the following formula (I): 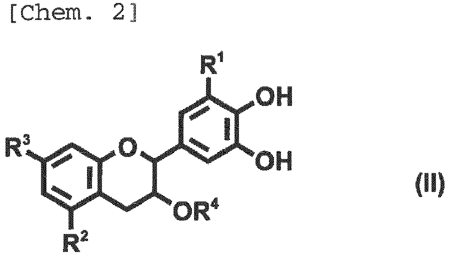 (in the formula, R¹, R² and R³ each independently represent a hydrogen atom or a hydroxy group, and R⁴ represents a hydrogen atom or a galloyl group.)
6. The supercooling promoting agent according to 5 above, in which the catechin-type tannin is a polyphenol selected from a group consisting of a lychee fruit oligomerized polyphenol (Oligonol, trade name of the lychee fruit oligomerized polyphenol, manufactured Amino Up Chemical Co., Ltd.), lychee fruit polyphenol (LFP), and grape seed polyphenol (GSP).
7. The supercooling promoting agent according to 6 above, in which the polyphenol is the lychee fruit oligomerized polyphenol (Oligonol) or the lychee fruit polyphenol and has a supercooling activity for ultrapure water.
8. The supercooling promoting agent according to 5 above, in which the catechin-type tannin is selected from catechins consisting of tea catechin, catechin (C), epicatechin (EC), epicatechin gallate (ECG), epigallocatechin (EGC), gallocatechin gallate (GCG), and epigallocatechin gallate (EGCG).
9. A nonfreezing liquid, which is obtained by dissolving the supercooling promoting agent according to any one of 1 to 8 above in water or an aqueous solution containing an additive appropriate for an application, in which a content of the supercooling promoting agent in the nonfreezing liquid is 0.01 to 30 g/L.
10. The nonfreezing liquid according to 9 above, in which the additive is a component of a medium for culture of animal or plant cells or a component of a solution for storage of a biological material.
11. The nonfreezing liquid according to 9 above, further including a freezing damage inhibitor at 1 to 40 vol%.
12. The nonfreezing liquid according to 11 above, in which the freezing damage inhibitor is one kind or two or more kinds selected from the group consisting of methanol, ethanol, acetamide, dimethyl sulfoxide (DMSO), formaldehyde, ethylene glycol, propylene glycol, glycerin, proline, glucose, sorbitol, sucrose, trehalose, polyethylene glycol, dextran 10-150, polyvinylpyrrolidone (PVP), albumin, Ficoll, and hydroxyethyl starch (HES).
13. The nonfreezing liquid according to 9 or 11 above, in which the nonfreezing liquid further includes a biological material and is cooled to 0 to -15°C.
14. A vitrification solution including a freezing damage inhibitor singly or in combination at 20 to 90 vol% and, as a balance, water or an aqueous solution containing an additive appropriate for an application, in which the vitrification solution further includes the supercooling promoting agent according to any one of 1 to 8 above at 0.01 to 30 g/L.
15. The vitrification solution according to 14 above, in which a content of the water or the aqueous solution containing an additive appropriate for an application is 40 to 80 vol%.
16. The vitrification solution according to 14 or 15 above, in which the vitrification solution further includes a biological material and is cooled to liquid nitrogen temperature.

### Advantageous Effects of Invention

The supercooling promoting agent of the present invention can lower the freezing temperature of water or a substance containing water by about 15°C compared with the original temperature at which water freezes. The supercooling promoting agent can supercool bulk water at low temperature stably for a long period of time. In addition, when the supercooling promoting agent of the present invention is mixed with water, a nonfreezing liquid which can be used at about -15°C is obtained. A biological material or the like can be stored in the nonfreezing liquid at low temperature for a long period of time. When the supercooling promoting agent of the present invention is dissolved in water, an aqueous solution containing an additive appropriate for its application, or a substance containing water, and frozen, the agent can be used as a freezing controlling agent for controlling the size of ice crystals. Addition of the supercooling promoting agent can reduce the size of ice crystals formed by a decrease in the freezing initiation temperature due to supercooling. Therefore, when the aqueous solution having added thereto the supercooling promoting agent is frozen after changing a cooling rate or changing the composition or concentration of the additive, the agent can be used as a freezing controlling agent for changing the size of ice to various degrees. Further, when the supercooling promoting agent of the present invention is added to a vitrification solution containing a freezing damage inhibitor at a high concentration, the concentration of the vitrification solution can be reduced to lower the toxicity provided by immersion into the vitrification solution. As a result, at ultralow temperature such as the liquid nitrogen temperature, a vitrified product can be provided efficiently, and a biological material or the like, which has hitherto been difficult to store in a vitrified product, can be stored in the vitrified product at ultralow temperature.

### Brief Description of Drawings

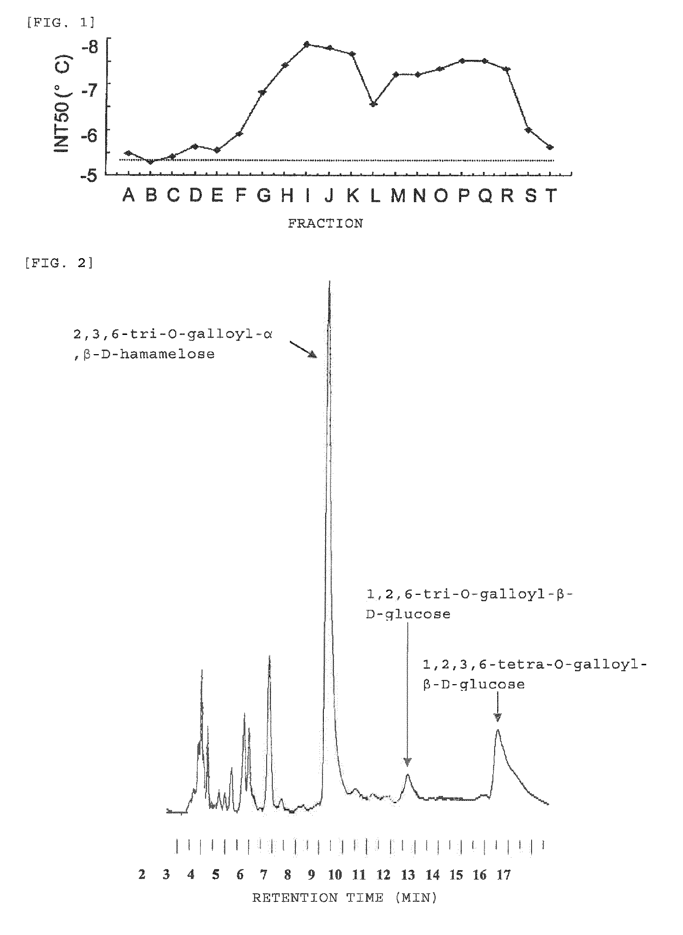

[FIG. 1] A graph showing supercooling activities of silica gel column chromatography fractions obtained from an ethyl acetate soluble fraction of an extract of *Cercidiphyllum japonicum.* The horizontal axis silica gel column chromatography fractions A to T, and the vertical axis shows supercooling activities (INT50 (°C)).
[FIG. 2] A high-performance liquid chromatogram of the fractions K to S.

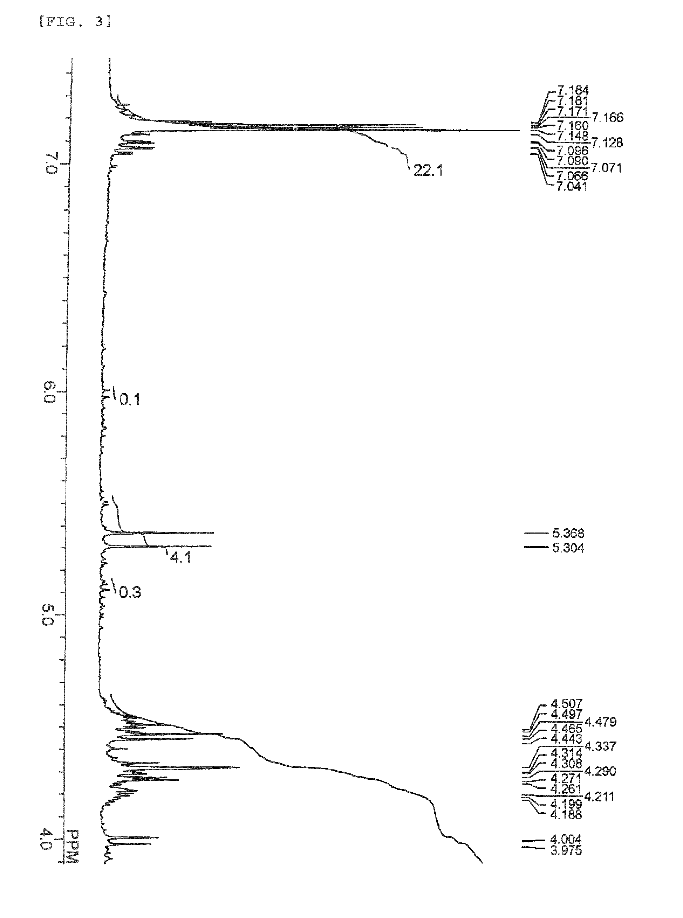

[FIG. 3] An ¹H-NMR spectrum of 2,3,6-tri-O-galloyl-α,β-D-hamamelose isolated from the fractions K to S (measured in deuterated acetone (acetone-d₆)).

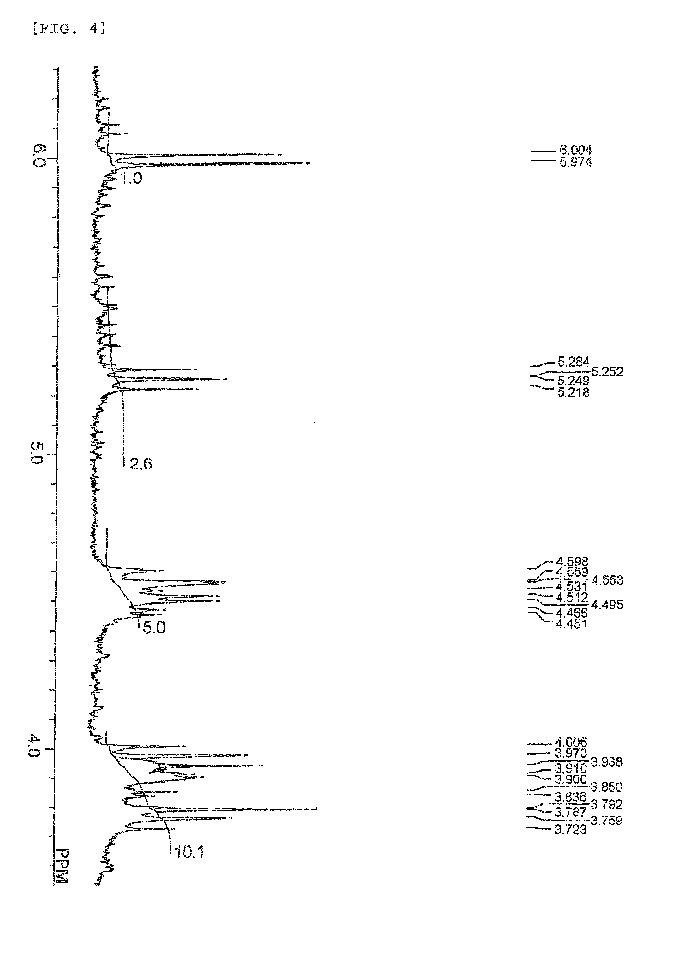

[FIG. 4] An ¹H-NMR spectrum of 1,2,6-tri-O-galloyl-β-D-glucose isolated from the fractions K to S (measured in deuterated methanol (CD₃OD)).

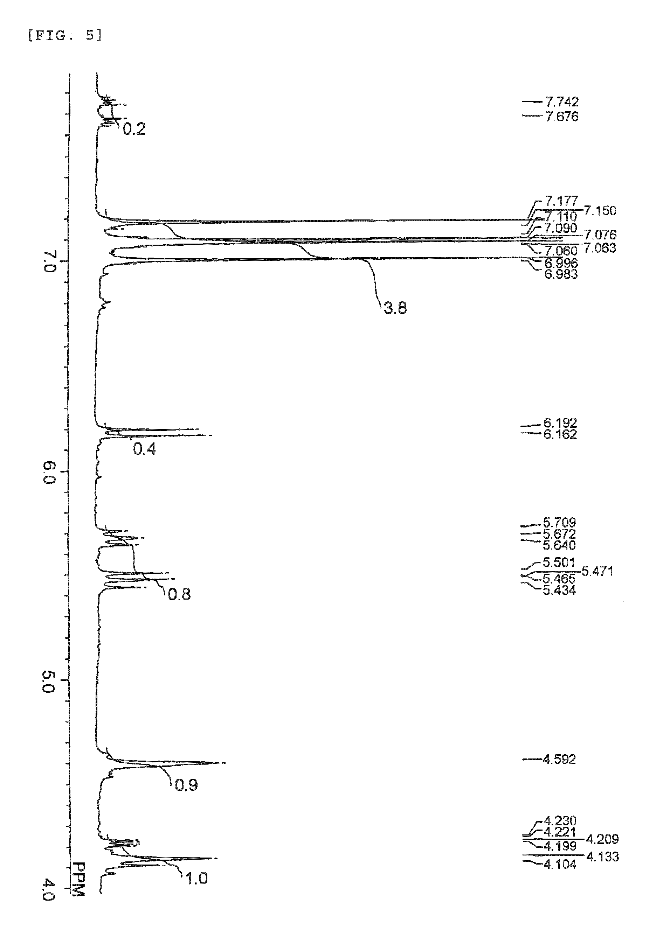

[FIG. 5] An ¹H-NMR spectrum of 1,2,3,6-tetra-O-galloyl-β-D-glucose isolated from the fractions K to S (measured in deuterated methanol (CD₃OD)).

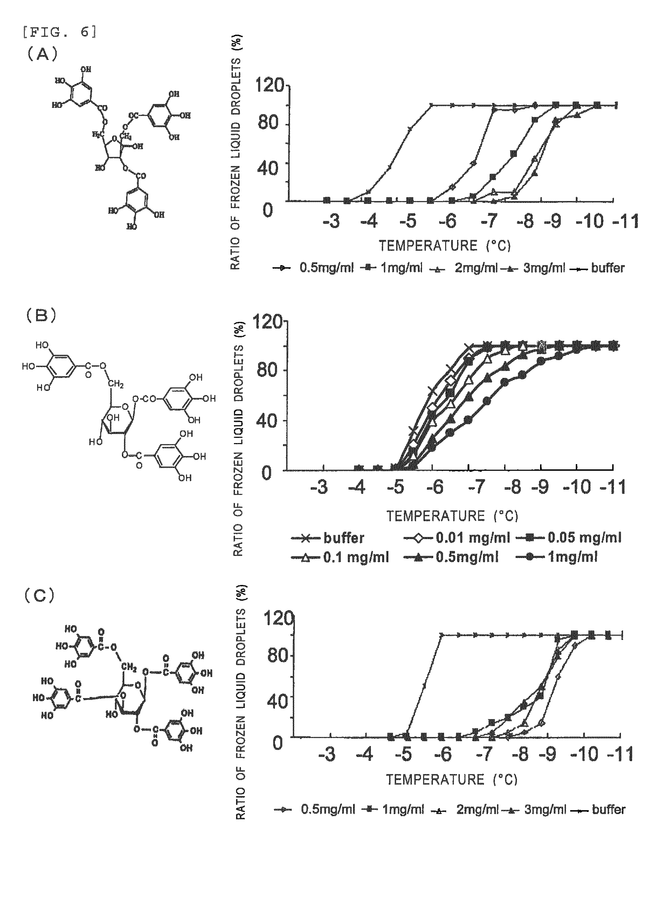

[FIGS. 6A, 6B, 6C] Graphs respectively showing the supercooling activities of 2,3,6-tri-O-galloyl-α,β-D-hamamelose, 1,2,6-tri-O-galloyl-β-D-glucose, and 1,2,3,6-tetra-O-galloyl-β-D-glucose each isolated from the fractions K to S, in which the horizontal axis indicates the temperature of a copper plate on which droplets are mounted and the vertical axis indicates a ratio of frozen droplets.

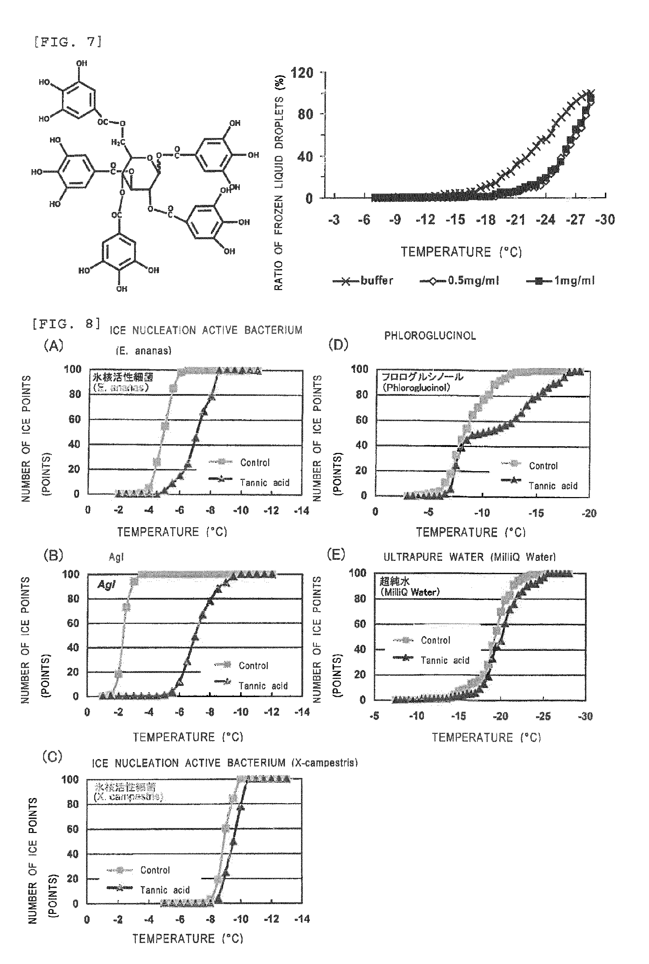

[FIG. 7] A graph showing the supercooling activity of a 1,2,3,4,6-pentagalloyl-α,β-D-glucopyranose mixture (α:β=1:3.8) prepared in Example 2.
[FIGS. 8] Graphs showing supercooling promoting effects of 0.1% tannic acid (nutgall) on various ice nucleus substances (A: ice nucleation active bacterium (*Erwinia ananas*), B: silver iodide (AgI), C: ice nucleation active bacterium (*Xanthomonas campestris*), D: phloroglucinol) and ultrapure water (MilliQ Water).

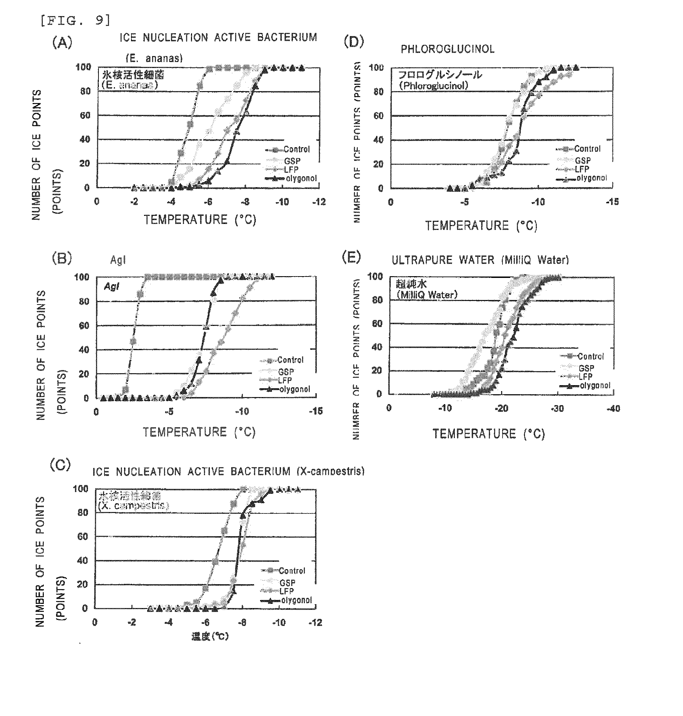

[FIGS. 9] Graphs showing supercooling promoting effects of 0.1% polyphenols on various ice nucleus substances (A: ice nucleation active bacterium (*Erwinia ananas*), B: silver iodide (AgI), C: ice nucleation active bacterium (*Xanthomonas campestris*)*,* D: phloroglucinol) and ultrapure water (MilliQ Water).

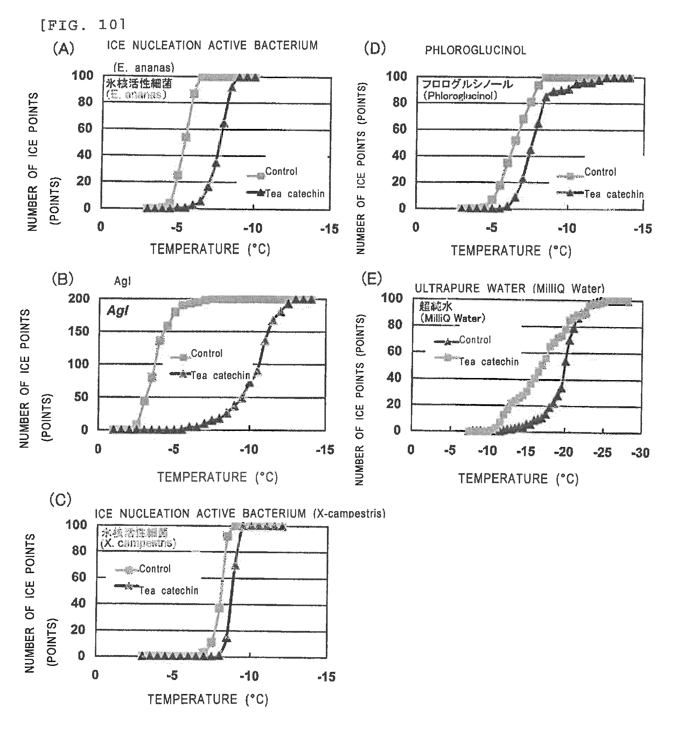

[FIGS. 10] Graphs showing supercooling promoting effects of 0.1% tea catechin on various ice nucleus substances (A: ice nucleation active bacterium (*Erwinia ananas*)*,* B: silver iodide (AgI), C: ice nucleation active bacterium (*Xanthomonas campestris*), D: phloroglucinol) and ultrapure water (MilliQ Water).

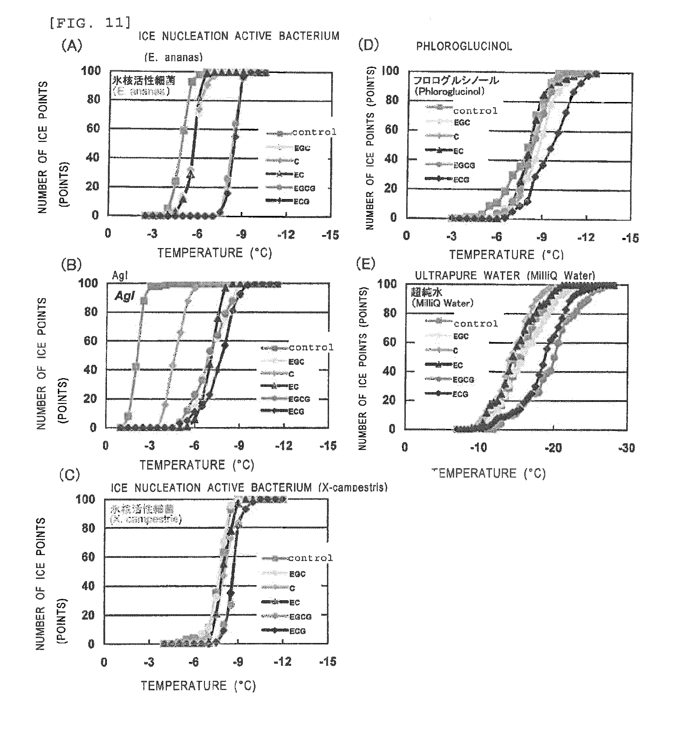

[FIGS. 11] Graphs showing supercooling promoting effects of 0.1% catechins onvarious icenucleus substances (A: ice nucleation active bacterium (*Erwinia ananas*), B: silver iodide (AgI), C: ice nucleation active bacterium (*Xanthomonas campestris*), D: phloroglucinol) and ultrapure water (MilliQ Water).

### Mode for Carrying out the Invention

A supercooling promoting agent comprising a tannin according to the present invention is contained in all organisms such as a tree and may be extracted from the organisms or biological substances or chemically synthesized. As the tree of interest, a tree which contains a supercooling promoting substance in a large amount and grows in a cold regions is suitable. Examples of such tree include: conifers such as Japanese Larch, *Thuja occidentalis,* Japanese yew, Japanese cedar, Nikko fir, Sakhalin fir, Jezo spruce, Glehn's spruce, *Pinus parviflora* var. *pentaphylla*, *Pinus strobus,* Japanese red pine, and Japanese black pine; and broad-leaved trees such as silver birch, aspen, Japanese chestnut, Japanese rowan, *Styrax obassia, Quercus mongoloca* var. *grosseseratta,* Japanese elm, and *Cercidiphyllum japonicum.* Regardless of the amount of the supercooling promoting substance, a tree which grows in a region other than the cold region may be used. The supercooling promoting substance in any such tree is considered to be present in living cells (parenchyma cells) but may be present in parts other than the cells. The substance is stable and can be extracted from not only a living tree but also a withered tree or wood stored for a long period of time. Tannins are extracted from not only woody portions including sapwood and heartwood of the tree species but also, for example, tree barks, winter buds, leaves, and other plant bodies.

As a raw material for obtaining a tannin or a plant extract containing the tannin from other plant bodies, the whole or parts of mature or immature fruits, fruit skins, seeds, leaves, stems, petiole, branches, roots, or flowers of a plant including the tannin or plant extract containing the tannin are used. The extracts obtained from those raw materials include dried products and pulverized products of plant bodies themselves, juices obtained by squeezing plant bodies themselves, crude extracts, purified products, and the like. Specific examples of the plant and processed plant products each containing a tannin include tea, persimmon, mimosa, quebracho, gambier, cassia, green tea, red tea, oolong tea, persimmon juice, Chinese nutgall, Turkishgallnut, Japanese angelica tree, Aleppo, myrobalan, sumac, *Geranium thunbergii,* grape seed, pine bark, buckwheat, lychee, wine, pigeon pea tea, acorn, Japanese chestnut, unripeapple fruit, cacao, and blueberry. However, aplant other than those described above may also be used as long as it contains a tannin.

A preparation method for the extract is not particularly limited. General means used for separating or purifying a tannin from a tree or any other plant body may be adopted. Examples of the general means include: fragmentation, freezing, pulverization, extraction, separation, concentration, and drying of a xylem tissue of a tree; extraction, concentration, filtration, liquid separation, fractional precipitation, crystallization, drying, vacuum drying, and lyophilization of a plant body; and fractionation, adsorption chromatography, reversed phase chromatography, hydrophobic interaction chromatography, hydrophilic interaction chromatography, gel filtration chromatography, ion exchange chromatography, thin layer chromatography, and high-performance liquid chromatography of a water-soluble fraction or organic solvent-soluble fraction of any crude extract, which are adopted as required. A fraction fractionated from an extract of interest can be obtained by a method selected from the conventional means. For example, the fraction may be obtained by: performing extraction for a pulverized product of a tree or another plant body containing a tannin with water, an organic solvent, or a mixed solvent thereof; and fractionating the resultant extract with an organic solvent by the various kinds of chromatography. The resultant fraction contains a tannin and is subjected to a necessary treatment such as concentration, purification, sterilization, or drying before use. Examples of the organic solvent to be used include ethyl acetate, methanol, ethanol, glycerin, 1,3-butylene glycol, chloroform, and dichloromethane. Extraction is usually carried out at room temperature or under heating at 100°C or less. The extract itself maybe concentrated or dried, or a supernatant separated and collected from the extract may be concentrated or dried. Thus, the tannin can be obtained as an extract of a tree or another plant body. In the case where the tannin contained in a tree, another plant body, and a plant-processed product is used as the supercooling promoting agent, it is not necessary to use a purified tannin, and an extracted fraction containing the tannin may be used.

A tannin is one of polyphenols distributed widely in the plant kingdom, and the tannin which is a natural extract from a crude drug has been used as an herb medicine such as an obstruent, an agent for tannage, or a metallic ion precipitating agent from older times. In general, tannins are broadly classified into hydrolyzable tannins and condensed tannins. The former is further classified into a gallotannin and an ellagitannin, and the latter is further classified into a simple condensed tannin and a complex condensed tannin. The gallotannin is hydrolyzed with an acid or the like into a polyvalent phenol acid and a polyvalent alcohol such as a sugar and mainly provides a polyphenol acid such as gallic acid, and the ellagitannin provides a polyhydric phenol such as a gallic acid dimer.

A typical example of the hydrolyzable tannin according to the present invention is a sugar ester-type hydrolyzable tannin having a partial structure in which one or more hydroxy groups of a sugar having 6 carbon atoms are each ester-bonded to a substituted benzoyl group selected from structures represented by the following formulae (1) to (6): 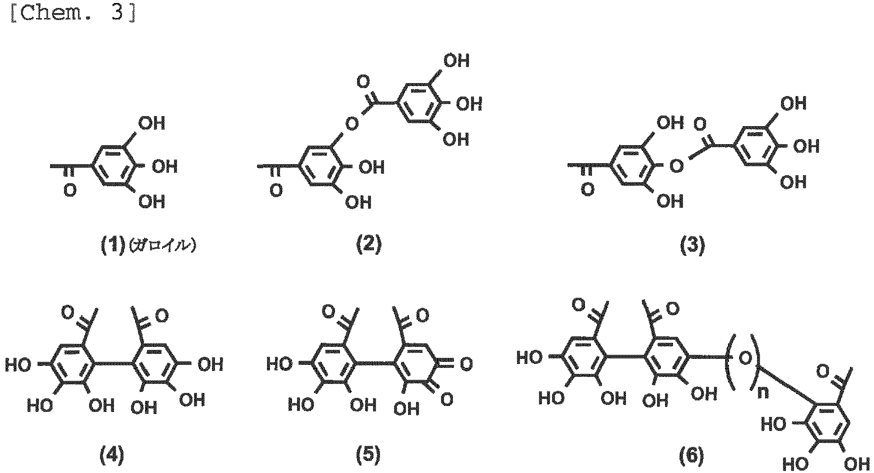 (wherein n represents 0 or 1).

Examples of the sugar having 6 carbon atoms include: ketohexoses such as psicose, fructose, sorbose, and tagatose; aldohexoses such as allose, altrose, glucose, mannose, gulose, idose, galactose, and talose; and hexoses such as deoxy sugars including fucose, fuculose, and rhamnose. As a representative example of those hydrolyzable tannins, there is given a sugar ester-type hydrolyzable tannin having a partial structure in which one or more hydroxy groups derived from a furanose represented by formula (Ia): 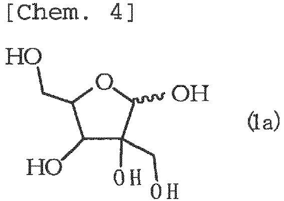 , and glucose represented by formula (Ib): 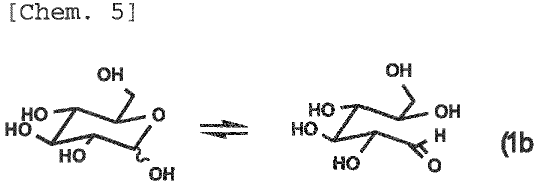 are each ester-bonded to any substituted benzoyl group selected from the structures represented by the formulae (1) to (6).
Preferred specific examples of the hydrolyzable tannin include galloylated monosaccharides such as castalagin, geraniin, geraniinic acids, kurigalin, sanguiin H-10, β-glucogallin, aceritannin, eugeniin, corilagin, trigalloylribofuranose, trigalloylglucose, trigalloylhamamelose, trigalloylglucose, tetragalloylglucose, pentagalloylglucose, and octagalloylglucose.

The gallotannin includes digalloylglucose, in which the galloyl group of galloylglucose is further bonded to another galloyl group with a depside bond, and an example thereof is tannic acid. Tannic acid is obtained from nutgall, gallnut, Chinese peony, tara, orthelike, and is commercially available as an official drug (Chinese gallotannin) or the like. Examples of the ellagitannin include geraniin contained in Geranium *thunbergii* of the family Geraniaceae, chebulinic acid contained in *Terminalia* chebula of the family Combretaceae, cornusiin A contained in *Cornus officinalis* of the family Cornaceae, and agrimoniin contained in *Agrimonia pilosa* the family Rosaceae.

As the condensed tannin, there is known a catechin-type tannin. The catechin-type tannin includes a compound containing, as apartial structure, a flavan-3-ol skeleton represented by formula (II): 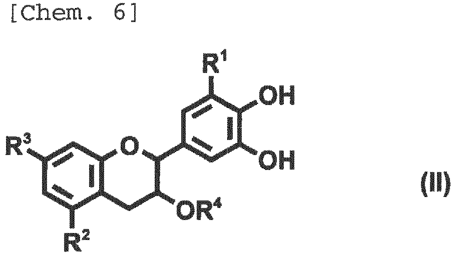 (where R¹, R², and R³ each independently represent a hydrogen atom or a hydroxy group, and R⁴ represents a hydrogen atom or a galloyl group).

In the catechin-type tannin containing, as a partial structure, the flavan-3-ol skeleton represented by formula (II), when the formula represents a catechin molecule, substituents at the 4-position and the 8-position are each substituted with a hydrogen atom, and when the formula represents a polycatechin, the 4-position and the 8-position are subjected to condensation. Specific examples thereof include: catechin molecules such as tea catechin, catechin gallate, catechin, epicatechin, epigallocatechin, epicatechin gallate, epigallocatechin gallate, and gallocatechin gallate; and polycatechins such as Oligonol, Flavangenol, rhatannin, 3-O-galloylprocyanidin B-1, procyanidins B-2 and C-1, cinnamtannins B1 and D1, proanthocyanidins, theaflavins, theasinensins, and thearubigins.

Molecules of polyphenols such as: gallic acid (GA) and the methyl ester thereof (methyl gallate: MG) and ellagic acid (EA) which are obtained by acid hydrolysis of gallotannin and ellagitannin; and chlorogenic acid (ChA) and caffeic acid (CaA) which are isolated from coffee beans are also found to have supercooling activities. For example, against an ice nucleus substance AgI, GA has a supercooling activity of -1.2°C, MG has a supercooling activity of -3.3°C, EA has a supercooling activity of -0.3°C, ChA has a supercooling activity of -0.6°C, and CaA has a supercooling activity of -1.9°C.

The hydrolyzable tannins have different galloylation degrees, which show numbers of pentagalloyl groups per 1 mol of galloyl glucose, depending on raw materials from which the tannins are derived and have different molecular weights. Specific examples of the raw materials preferably include nutgall (such as Chinese nutgall), gallnut (such as Turkish gallnut), tara, aleppo, myrobalan, and sumac.

In the condensed tannin, two or more catechin molecules are generally condensed with carbon-carbon bonds at their 4- and 8-positions. As a raw material for the condensed tannin, there may be preferably used tea, persimmon, mimosa, quebracho, gambier, cassia, grape seed, pine bark, and litchee. Further, in the case of a high-molecular-weight tannin, an oligomer prepared using the tannin as a raw material so as to have a low molecular weight may be used.

The supercooling promoting substance comprising a tannin according to the present invention inhibits formation of ice nuclei at a low concentration regardless of freezing-point depression although a conventional antifreeze solution such as ethylene glycol inhibits the formation based on concentration-dependent molar freezing-point depression. That is, in general, when the supercooling promoting agent according to the present invention is added to water at a low concentration of 1 vol% or less or 1 mass% or less, the agent exhibits a supercooling activity significantly superior to the concentration-dependent freezing-point depression. General substances such as salts, sugars, and sugar alcohols promote the supercooling activity about twice the freezing-point depression temperature, while the supercooling promoting substance according to the present invention promotes the supercooling activity ten times or more, in some cases, 100 times or more. Meanwhile, conventional antifreeze proteins inhibit growth of ice crystals obtained by formation of ice nuclei and do not inhibit the formation of ice nuclei itself. On the other hand, the supercooling promoting substance according to the present invention inhibits the formation of ice nuclei itself.

In general, freezing of water is considered to relate to an ice nucleus-forming bacterium and a residue thereof as ice nucleus-forming substances. In the present invention, dead bacterial cells of two kinds of living organism-derived bacteria (ice nucleation active bacteria (*Erwinia ananas* and *Xanthomonas campestris*)) and two kinds of non-living matter-derived substances (silver iodide (AgI) and phloroglucinol)) were used as the ice nucleus-forming substances to measure and evaluate supercooling activities of tannins.

The supercooling promoting agent comprising a tannin of the present invention interacts with the ice nucleus-forming substances to exhibit supercooling activities of -0.1 to -6.8°C against the ice nucleus-forming substances and to exhibit supercooling activities of -0.3 to -4.5°C against ultrapure water (MilliQ Water) . In particular, tannic acid, Oligonol, lychee fruit polyphenol (LFP), epigallocatechin gallate (EGCG), andepicatechin gallate (ECG) exhibit satisfactory supercooling activities against all of the ice nucleus substances and pure water and are practically excellent as the supercooling promoting agents. Freezing of water occurs with various foreign substances inwater serving as icenuclei. The foreign substances are of great variety. Further, from a practical standpoint, there is a demand for a supercooling activity against normal water containing various ice nucleus-forming substances which cannot be identified (including pure water) . Any tannin selected from tannic acid, Oligonol (trade name of lychee fruit oligomerized polyphenol manufactured by Amino Up Chemical Co., Ltd.), LFP, EGCG, GCG, and ECG can supercool water and is superior to flavonoid glycosides whose supercooling activities vary depending on the type of water to be supercooled. Further, water having added thereto a tannin supercools even water which generates a large amount of air bubbles at -10°C for a few days.

Further, the supercooling promoting effect of the tannin according to the present invention on ultrapure water is superior to that of a flavonoid glycoside. That is, tannic acid exhibits a supercooling activity of -3.7°C against ultrapure water (MilliQ Water), and Oligonol, EGCG, and ECG exhibit excellent supercooling activities of -3.2 to -4.5°C against ultrapure water (MilliQ Water), while the flavonoid glycoside exhibits little supercooling activity. Therefore, the tannin is effective in supercooling of pure water compared with the flavonoid glycoside and can be used for preventing freezing of a large volume of water (such as water for fire-fighting in an oil storage station), and hence the tannin can be used as the supercooling promoting agent. The tannin can be prepared very inexpensively in a large amount compared with the flavonoid glycoside, and is easily dissolved inwater compared with the flavonoid glycoside. Therefore, a wide variety of drinking water, drugs, and the like which can be drunk at -10°C can be prepared.

In addition, the supercooling promoting agent according to the present invention has not only excellent supercooling promoting effects of -2.6 to -6.8°C on AgI but also good supercooling promoting effects of -0.8 to -3.5°C on *Erwinia ananas,* which is known to be an ice nucleation active bacterium having an ice nucleation function at relatively high temperatures (e.g., -6 to 0°C) (see, for example, JP 2000-106868 A) . Inparticular, tannic acid, Oligonol, LFP, and tea catechin exhibit good supercooling activities of -2.2 to -2.7°C against *Erwinia ananas,* and EGCG and ECG, which are catechins, exhibit excellent supercooling activities of -3.4 to -3.5°C against *Erwinia ananas.*

Further, the tannin according to the present invention has an excellent supercooling activity compared with those of other so-called supercooling promoting substances as mentioned below.
1) Unidentified crude extracts extracted from seeds of various plants (including peach) exhibit supercooling activities of -2.6 to -8.1° against water (Caple et al., (1983) Cryoletters, 4, 59-64.). However, the values were obtained using only silver iodide which has low ability as an ice nucleus-forming substance at a cooling rate of 1°C/min which is significantly higher than the cooling rate of the supercooling promoting agent of the present invention (0.2°C/min) and is a condition where temporal supercooling easily occurs.
2) Eugenol extracted from clove and a similar substance thereof exhibit supercooling activities of -0.2 to -2.5°C against water (Kawahara and Obata (1996) J. Antibact. Antifung. Agents, 24, 95-100.). The concentration of the substance added is 1 mg/mL, and the cooling rate is 1°C/min, which is significantly higher than that of the supercooling promoting agent of the present invention and is a condition where temporal supercooling easily occurs.
3) Hinokitiol and a similar substance thereof exhibit supercooling activities of -0.4 to -2.1°C against water (Kawahara et al., (2000) Biosci. Biotechnol. Biochem., 64, 2651-2656.). The concentration of the substance added is 10 mM, and the cooling rate is 1°C/min, which is significantly higher than that of the supercooling promoting agent of the present invention and is a condition where temporal supercooling easily occurs.
4) A chitinpolysaccharide (130kDa) extracted from a bacterium exhibits supercooling activities of 0 to -4.2°C against water (Yamashita et al., (2002) Biosci. Biotechnol. Biochem., 66, 948-954). The concentration of the substance added is 50 µg/mL, and the cooling rate is 1°C/min, which is significantly higher than that of the supercooling promoting agent of the present invention and is a condition where temporal supercooling easily occurs.
5) Various antifreeze proteins exhibit supercooling activities of a maximum of -7.8°C against water (Duman (2002) J. Comp. Physiol., 172, 163-168.). However, the concentration of the antifreeze protein added at which the maximum value was obtained is unclear, and the value was obtained when a high concentration (0.5 M) of citric acid was added. In the case of using only the antifreeze protein, supercooling is promoted only by -1.2°C.

The supercooling promoting agent including a tannin of the present invention may be used as a nonfreezing liquid by being dissolved in water usually at 0.01 g/L or more, preferably 0.01 to 30 g/L, more preferably 0.01 to 10 g/L, still more preferably 0.1 to 1.0 g/L. The nonfreezing liquid is usually obtained by dissolving the tannin in water, but an aqueous solution containing an additive appropriate for its application may be used instead of water. Examples of the additive include a component of a medium for culture of animal or plant cells and a component of a solution for storage of biological materials. The concentration of the additive in the aqueous solution may be appropriately determined depending on its application.

The nonfreezing liquid may further include another supercooling promoting agent or a freezing damage inhibitor. In the case where the nonfreezing liquid includes the freezing damage inhibitor, the liquid may include the freezing damage inhibitor singly or in combination at 1 to 40 vol%, preferably 1 to 20 vol%. The freezing damage inhibitor refers to a substance for reducing damage due to freezing by being added to a biological material or an aqueous solution where the material is immersed. Any substance called freezing damage inhibitor has one, or a combination of two or more, of an effect of causing concentration-dependent freezing-point depression, an effect of reducing the amount of ice crystals formed, an effect of reducing an increase in a salt concentration in a frozen material, an effect of facilitating vitrification, and the like. Examples of such freezing damage inhibitor include methanol, ethanol, acetamide, dimethyl sulfoxide (DMSO), formaldehyde, ethylene glycol, propylene glycol, glycerin, proline, glucose, sorbitol, sucrose, trehalose, polyethylene glycol, dextran 10-150, polyvinylpyrrolidone (PVP), albumin, Ficoll, and hydroxyethyl starch (HES).

In the case where the nonfreezing liquid includes no freezing damage inhibitor or in the case where the nonfreezing liquid includes an additive such as the freezing damage inhibitor at a concentration which hardly affects the freezing-point depression (about 1 mass% or less), a liquid state can be maintained in temperature as low as about -15°C for a long period of time (1 to 2 weeks). When biological materials (e.g., plant or animal cells or tissues, edible or ornamental fish and shellfish, plants themselves such as vegetables, or parts thereof) are immersed into the nonfreezing liquid and cooled, the materials can be stored at a low temperature of 0°C or less, particularly in a temperature range of about 0 to -15°C for a long period of time without causing freezing although the liquid is usually used at a low temperature of about 5°C or less. The nonfreezing liquid can reduce the size of ice crystals because a freezing initiation temperature is lowered by supercooling. Further, when the non freezing liquid is used singly or in combination with a freezing damage inhibitor or the like, the liquid can be used as a freezing controlling agent for, for example, medicines and foods prepared by freeze-drying. Also in an extract (such as a crude extract) from a biological material such as a tree containing the above-mentioned substance, the nonfreezing liquid can be applied in the same way as above.

On the other hand, an aqueous solution containing the above-mentioned freezing damage inhibitor at a high concentration is called "vitrification solution," and water becomes a vitrified product (amorphous ice) without forming crystals even at ultralow temperature (for example, liquid nitrogen temperature) ("Manual of plant storage at ultralow temperature" edited by Takao Niino et al., published by National Institute of Agrobiological Sciences, 2006). The vitrification solution refers to a solution containing the freezing damage inhibitor singly or in combination of two or more thereof at 20 to 90 vol%, preferably 40 to 90 vol% and water as a balance. As the water, a solvent such as a medium for culture of animal or plant cells may be used. In the case where the solution is used for culture or storage of animal or plant cells, water or the medium for culture of animal or plant cells is mixed therein preferably at 30 vol% or more, particularly preferably at 40 vol% or more. PVS2 which is currently the most widely used vitrification solution is obtained by adding 30 vol% of glycerin, 15 vol% of ethylene glycol, 15 vol% of DMSO, and 0.4M of sucrose to a medium solution. The kind and concentration of the medium solution are appropriately changed depending on materials to be cultured or stored. In the present invention, the supercooling promoting agent of the present invention (the above-mentioned tannin) is added to the vitrification solution usually at 0.01 g/L or more, preferably 0.01 to 30 g/L, more preferably 0.01 to 10 g/L, still more preferably 0.1 to 1.0 g/L. Such vitrified product can be maintained in an amorphous state at a temperature equal to or lower than the freezing temperature of the vitrification solution, for example, at -15°C or less, particularly in a temperature range of -60 to -273°C, for example, at the liquid nitrogen temperature (77 K).

In freeze storage by vitrification, a material to be stored is usually subjected to an immersion treatment at room temperature or at a temperature of 0°C or higher for a short period of time. According to the pre-freezing treatment, water in the material is removed by a high concentration of the vitrification solution, and water in the material is replaced by the vitrification solution. Therefore, when the material is fed to liquid nitrogen, water present in or outside the material is vitrified without forming ice crystals. When a biological material such as a plant is added to the vitrification solution and fed to liquid nitrogen, water present in or outside the biological material becomes a vitrified product (amorphous ice) . The material in a vitrified state is not damaged by freezing, and hence the biological material can be frozen and stored in the vitrification solution at ultralow temperature.

### Examples

Hereinafter, the present invention is specifically described by way of examples, However, these examples are not intended to limit the present invention.
In the present invention, a supercooling activity (also referred to as ice nucleus formation inhibitory activity) was measured by the following method. That is, an object to be measured was mixed at 0.5 mg/mL in a buffer containing an ice nucleus substance to prepare a solution, and many 2-µL liquid droplets of the solution were placed on a copper plate where the temperature could be controlled. The copper plate was cooled at 0.2°C/min, and the number of frozen liquid droplets was visually observed. The temperature at which 50% of the liquid droplets were frozen was defined as a freezing temperature, and a difference between the freezing temperature of the solution including the object to be measured and the ice nucleus substance and the freezing temperature of a solution including only the ice nucleus substance and the buffer (control) (INT50 (°C)) was defined as a supercooling activity. As the buffer, a 50 mM potassium phosphate buffer (pH 7.0) was used.

### Example 1:

Branches were collected from *Cercidiphyllum japonicum* which grew in Sapporo, Hokkaido. Xylem tissues of the branches of *Cercidiphyllum japonicum* were fragmented by a pencil sharpener, frozen in liquid nitrogen, and pulverized as finely as possible using a mortar and a pestle. The resultant pulverized product of the xylem tissues (3.7 Kg) was immersed in 20 L of methanol for 2 weeks . The resultant extract was centrifuged at 14, 000 G (Hitachi : HIMC CF15R), and the supernatant was collected. The supernatant was dried, and 93.8 g of the dried product was dissolved in 300 mL of water.
The water suspension of the crude extract was centrifuged at 20°C and 14,000 G, and the supernatant was collected. 300 mL of the supernatant and 600 mL of ethyl acetate were mixed, and the mixture was divided into a water-soluble part and an ethyl acetate-soluble part by a separating funnel, followed by drying. Supercooling activities of the parts were measured by the above-mentioned method. Dead bacterial cells of an ice nucleation active bacterium (*Erwinia ananas*) (manufactured by Wako Pure Chemical Industries, Ltd.) were used as an ice nucleus substance. The water-soluble part was found to have a supercooling activity of about -2°C, and the ethyl acetate-soluble part was found to have a supercooling activity of about -4°C.

The dried ethyl acetate-soluble fraction which exhibited the higher supercooling activity was divided into 20 fractions from A to T by self-made silica gel column chromatography with "hexane · 2-propanol · water" and "chloroform · methanol · water." Subsequently, for each of the fractions A to T, a supercooling activity (INT50 (°C)) was measured by the above-mentioned method. FIG. 1 shows the results.

The fractions K to S obtained above were analyzed by high-performance liquid chromatography (column: PC hydrophilic interaction chromatography column (HILIC), solvent: acetonitrile:water=9:1, flow rate: 1mL/min, detector: UV 280 nm) . FIG. 2 shows the results. 2, 3, 6-Tri-O-galloyl-α, β-D-hamamelose, 1, 2, 6-tri-O-galloyl-β-D-glucose, and 1, 2, 3, 6-tetra-O-galloyl-β-D-glucose shown in FIG. 2 were each isolated, and it was confirmed that the compounds each exhibited a supercooling activity. The three substances are known substances, and their structures were confirmed by ¹H-NMR. FIGS. 3 to 5 show the resultant ¹H-NMR charts. FIGS. 6 show the supercooling activity of water containing each of the three substances with 2 mg/mL *Erwinia ananas* serving as an ice nucleus-forming substance, together with control data in the case of containing none of the three substances. FIGS. 6(A) and 6(C) show data in the cases of containing 2, 3, 6-tri-O-galloyl-α, β-D-hamamelose and 1, 2, 3, 6-tetra-O-galloyl-β-D-glucose, respectively, each at 0.5 mg/mL, 1 mg/mL, 2 mg/mL, and 3 mg/mL, and FIG. 6(B) shows data in the cases of containing 1, 2, 6-tri-O-galloyl-β-D-glucose at 0.01 mg/mL, 0.05 mg/mL, 0.1 mg/mL, 0.5 mg/mL, and 1 mg/mL. The results have confirmed that each of kurigalin (2, 3, 6-tri-O-galloyl-α, β-D-hamamelose), 1, 2, 6-tri-O-galloyl-β-D-glucose, and galloylglucose (1, 2, 3, 6-tetra-O-galloyl-β-D-glucose), being a kind of hydrolyzable tannin, is identified in a methanol extract from the xylem of *Cercidiphyllum japonicum* having xylem parenchyma cells capable of undergoing deep supercooling to -40°C, and has an activity that allows water to be supercooled by about 5°C. It should be noted that kurigalin (2, 3, 6-tri-O-galloyl-α,β-D-hamamelose) is mainly contained in the fractions K to N, 1, 2, 6-tri-O-galloyl-β-D-glucose is mainly contained in the fractions N to O, and galloylglucose (1, 2, 3, 6-tetra-O-galloyl-β-D-glucose) is mainly contained in the fractions P to Q.

### Example 2:

According to Tetrahedron, 53, 10725-10732 (1997), a 1 ,2, 3, 4, 6-pentagalloyl-α, β-D-glucopyranose mixture (α:β=1:3.8) was prepared, and the supercooling activity was measured by the above-mentioned method. FIG. 7 shows the results.

### Example 3:

1 mL of distilled water containing no ice nucleus forming substance or tap water was placed in a test tube, and the test tube was placed at -10°C under vibration so that the water was constantly mixed with air to continuously generate air bubbles in the water. Those types of water instantly froze, but when 0.1% tannic acid (nutgall-derived), various 0.1% polyphenols (Oligonol (trade name, manufactured by Amino Up Chemical Co., Ltd.) , grape seed polyphenol (GSP, manufactured by Amino Up Chemical Co., Ltd.), lychee fruit polyphenol (LFP, manufactured by Amino Up Chemical Co., Lid.), 0.1% tea catechin, various 0.1% catechins (epigallocatechin (EGC), catechin (C), epigallocatechin gallate (EGCG), and epicatechin gallate (ECG)) were added at concentrations of 0.001% (W/W) or more to those types of water, the resultant mixtures continued supercooling for 3 days or more.

### Examples 4 to 8:

In Examples 4 to 8 below, 10 mm silver iodide (AgI) (manufactured by ACALAI TESQUE, INC.), 2 mg/mL dead bacterial cells of an ice nucleation active bacterium *Erwinia ananas* (manufactured by Wako Pure Chemical Industries, Ltd.), 2 mg/mL dead bacterial cells of an ice nucleation active bacterium *Xanthomonas campestris* (manufactured by Wako Pure Chemical Industries, Ltd.), and 120 mM phloroglucinol (manufactured by TOKYO CHEMICAL INDUSTRY CO., LTD.) were used as ice nucleus substances, and ultrapure water obtained by passing water through an ultrapure water device manufactured by Millipore Corporation was used (MilliQ Water). A substance to be measured (1 mg/mL) was mixed in a buffer containing any one of the ice nucleus substances and ultrapure water (MilliQ Water), and the supercooling activities were measured by the above-mentioned method. A difference between the freezing temperature of the solution containing the substance to be measured an the ice nucleus substance and the freezing temperature of the solution including only the ice nucleus substance, and a difference between the freezing temperature of pure water having dissolved therein the substance to be measured and the freezing temperature of only pure water were determined as supercooling activities (ice nucleus formation inhibitory activities) (see FIGS. 8 to 11 and Tables 1 to 4).

### Example 4:

Supercooling promoting effects were measured in the case of using the above-mentioned four kinds of ice nucleus substances at the above-mentioned concentrations and using, as a hydrolyzable tannin, tannic acid derived from insect galls (nutgalls) of *Rhus javanica*, available from Wako Pure Chemical Industries, Ltd., at a concentration of 0.1 mass%. FIGS. 8 (A) to 8 (E) show the results, and Table 1 shows freezing temperatures (INT50 (°C)) and supercooling activities (°C). The tannic acid derived from the nutgalls exhibited supercooling activities of -0.6 to -4.7°C against the ice nucleus substances (*E. ananas, X*. campestris, AgI, phloroglucinol) and ultrapure water (MilliQ Water) and a supercooling activity of -1.0°C against ultrapure water.

[Table 1]

**Table 1**

| | *Erwinia ananas* E. ananas | AgI | *Xanthomonas campestris* X.campestris | Phloroglucinol | Ultrapure water MilliQ Water |
|---|---|---|---|---|---|
| Tannic acid (°C) | -7.1 | -7.0 | -9.5 | -9.5 | -20.3 |
| Control (°C) | -4.9 | -2.3 | -8.9 | -8.3 | -19.3 |
| Supercooling activity (°C) | -2.2 | -4.7 | -0.6 | -1.2 | -1.0 |

### Example 5:

Various polyphenols: grape seedpolyphenol (GSP: manufactured by Layn, China), lychee fruit polyphenol (LFP: manufactured by Layn, China), and Oligonol (trade name of a lychee fruit oligomerized polyphenol manufactured by Amino Up Chemical Co., Ltd.) were added as condensed tannins at a concentration of 0.1mass to water containing the same four kinds of ice nucleus substances as above (*E. ananas, X*. *campestris,* AgI, and phloroglucinol) at above-mentioned concentrations and ultrapure water (MilliQ Water) , and supercooling promotingeffectsweremeasured. FIGS. 9 (A) to 9 (E) show the results. FIGS. 9 show that Oligonol and LFP exhibit supercooling activities against all of the ice nucleus substances and ultrapure water and have particularly excellent supercooling promoting effects on ultrapure water.
Table 2 shows the freezing temperatures (INT50 (°C)) and supercooling activities (°C).

[Table 2]

**Table 2**

| | *Erwinia ananas* E. ananas | AgI | *Xanthomonas campestris* X. campestris | Phloroglucinol | Ultrapure water MilliQ Water |
|---|---|---|---|---|---|
| Oligonol (°C) | -7.6 | -7.3 | -7.8 | -8.8 | -22.1 |
| LFP (°C) | -7.1 | -8.7 | -7.9 | -8.7 | -20.5 |
| GSP (°C) | -6.1 | -7.3 | -7.8 | - | - |
| Control (°C) | -4.9 | -2.5 | -6.7 | -7.9 | -18.9 |
| Supercooling activity (°C) | -1.2 to -2.7 | -4.8 to -6.2 | -1.1 to -1.2 | -0.8 to -0.9 | -1.6 to -3.2 |

The tannins (polyphenols) according to the present invention exhibited supercooling activities of -0.8 to -6.2°C against the four kinds of the ice nucleus substances. In particular, Oligonol and LFP exhibited supercooling activities of -0.8 to -6.2°C against the various ice nucleus substances and ultrapure water and excellent supercooling activities of -1.6 to -3.2°C against ultrapure water.

### Example 6:

Teacatechin (a product sold by GENRYOYA as a health supplement) was added as a catechin at a concentration of 0.1 mass% to water containing the four kinds of ice nucleus substances (*E*. *ananas, X*. *campestris,* AgI, and phloroglucinoly and ultrapure water (MilliQ Water), and the supercooling promoting effects were measured. FIGS. 10 (A) to 10 (E) show the results. FIGS. 10 show that the tea catechin has supercooling promoting effects on all of the ice nucleus substances (*E*. *ananas, X. campestris,* AgI, and phloroglucinol). In addition, Table 3 show the freezing temperatures (INT50 (°C)) and supercooling activities (°C).

[Table 3]

**Table 3**

| | *Erwinia ananas* E. ananas | AgI | *Xanthomonas campestris* x.campestris | Phloroglucinol |
|---|---|---|---|---|
| Tea catechin (°C) | -7.8 | -10.6 | -8.8 | -7.6 |
| Control (°C) | -5.4 | -3.8 | -8.1 | -6.4 |
| Supercooling activity (°C) | -2.4 | -6.8 | -0.7 | -1.2 |

As shown in Table 3, the catechin (tea catechin) according to the present invention exhibited supercooling activities of -0. 7 to -6.8°C against the four kinds of the ice nucleus substances,

### Example 7:

Epigallocatechin (EGC), catechin (C), epicatechin (EC), epigallocatechin gallate (EGCG), gallocatechin gallate (GCG), and epicatechin gallate (ECG) were added as catechins at a concentration of 0.1 mass% to water containing the four kinds of the ice nucleus substances (*E. ananas, X. campestris,* AgI, and phloroglucinol) and ultrapure water (MilliQ Water), and the supercooling promoting effects were measured. FIGS. 11 (A) to 11 (E) show the results. It should be noted that all of the catechins were purchased from Wako Pure Chemical Industries, Ltd. FIGS. 11 show that EGCG and ECG have supercooling promoting effects on all of the ice nucleus substances *(E. ananas, X. campestris,* AgI, and phloroglucinol) and ultrapure water (MilliQ Water) and particularly excellent supercooling promoting effects on ultrapure water (MilliQ Water).
In addition, Table 4 show the freezing temperatures (INT50 (°C) ) and supercooling activities (°C).

[Table 4]

**Table 4**

| | *Erwinia ananas* E.ananas | AgI | *Xanthomonas campestris* X. campestris | Phloroglucino 1 | Ultrapure water MilliQ Water |
|---|---|---|---|---|---|
| ECG (°C) | -8.4 | -7.8 | -8.7 | -9.6 | -18.7 |
| EGCG (°C) | -8.3 | -6.9 | -8.9 | -8.4 | -20.0 |
| EC (°C) | -5.7 | -7.1 | -7.9 | -8.1 | - |
| C (°C) | -5.7 | -4.7 | -8.1 | -8.3 | - |
| EGC (°C) | -5.7 | -6.9 | - | -8.8 | -15.8 |
| Control (°C) | -4.9 | -2.1 | -7.8 | -8.0 | -15.5 |
| Supercooling activity (°C) | -0.8 to -3.5 | -2.6 to -5.7 | -0.1 to -1.1 | -0.1 to -1.6 | -0.3 to -4.5 |

As shown in Table 4, the catechins according to the present invention exhibited supercooling activities of -0.1 to -5.7°C against the four kinds of the ice nucleus substances. In particular, ECG and EGCG exhibited supercooling activities of -0.4 to -5.7°C against the four kinds of the ice nucleus substances and excellent supercooling activities of -3.2 to -4.5°C against ultrapure water (MilliQ Water).

### Industrial Applicability

When the supercooling promoting agent of the present invention is added to a solution, the agent canbe used as anonfreezing liquid or a vitrification solution. When biological materials such as plant or animal cells or tissues, edible fish and shellfish, or vegetables are immersed in the nonfreezing liquid and cooled, the materials can be stored for a long period of time without causing freezing at a low temperature of 0°C or less, particularly in a temperature range of 0 to -15°C. Further, the agent can be applied as a supercooled drink. For example, storage of fresh foods such as fish and meat, import of food materials such as juices (if the materials are not frozen, an energy saving of 80 cal/g canbe achieved) or the like can be carried out by supercooling storage instead of freeze storage. In addition, the liquid can be applied to organ storage as an organ storage liquid used in organ transplantation or the like. The liquid can also be used as a coolant alternative to a petroleum-based coolant, such as a coolant for a large-scale computer or car engine. For the purpose of, for example, preventing frost formation in a freezer, preventing fog in a car window, or preventing dropwise condensation in tunnel, freezing may be prevented by coating the surface of the material with the liquid. When low-temperature resistance is imparted to a plant, a plant which can grow without freezing even at temperature below freezing can be created. When the liquid is spread to cloud, the amount of snowfall can be adjusted by suppressing formation of ice crystals. In addition, the liquid can be applied to freeze storage as a freezing controlling agent. The liquid can equalize uses of electricity by an ice thermal starage/transportation system.

## Claims

1. A supercooling promoting agent comprising a tannin.

2. The supercooling promoting agent according to claim 1, in which the tannin is a sugar ester-type hydrolyzable tannin having a partial structure in which one or more hydroxy groups of a sugar having 6 carbon atoms are each ester-bonded to a substituted benzoyl group selected from structures represented by the following formulae (1) to (6): 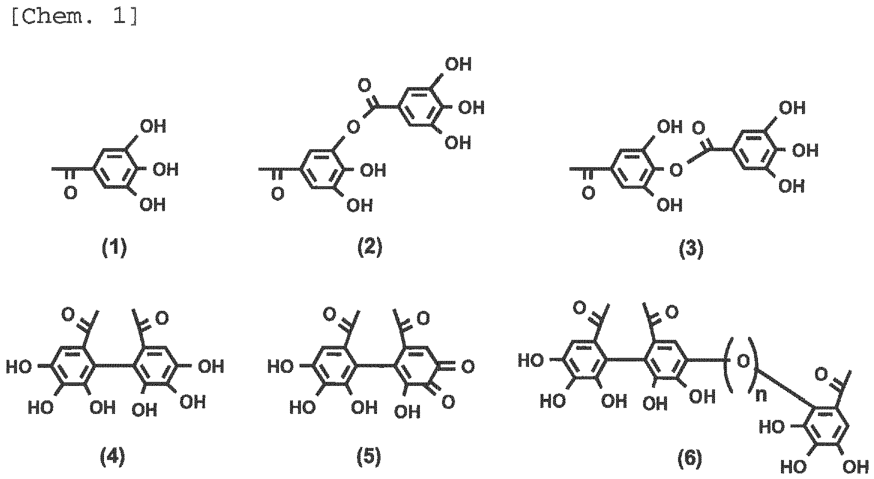 (wherein, n represents 0 or 1).

3. The supercooling promoting agent according to claim 2, in which the hydrolyzable tannin is 2, 3, 6-tri-O-galloyl-α, β-D-hamamelose, 1, 2, 6-tri-O-galloyl-β-D-glucose, or 1, 2, 3, 6-tetra-O-galloyl-β-D-glucose.

4. The supercooling promoting agent according to claim 2, in which the hydrolyzable tannin is tannic acid derived from nutgall.

5. The supercooling promoting agent according to claim 1, in which the tannin is a catechin-type tannin containing, as a partial structure, a flavan-3-ol skeleton represented by the following formula (I): 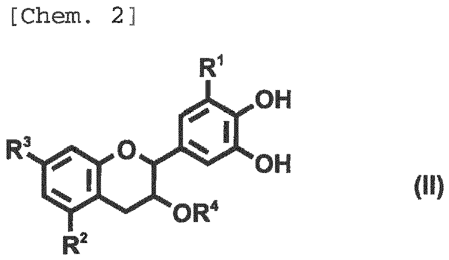 (Wherein R¹, R², and R³ each independently represent a hydrogen atom or a hydroxy group, and R⁴ represents a hydrogen atom or a galloyl group.)

6. The supercooling promoting agent according to claim 5, in which the catechin-type tannin is a polyphenol selected from a group consisting of a lychee fruit oligomerized polyphenol (Oligonol, trade name of the lychee fruit oligomerized polyphenol, manufactured Amino Up Chemical Co., Ltd.), lychee fruit polyphenol (LFP) , and grape seed polyphenol (GSP).

7. The supercooling promoting agent according to claim 6, in which the polyphenol is the lychee fruit oligomerized polyphenol (Oligonol) or the lychee fruit polyphenol and has a supercooling activity for ultrapure water.

8. The supercooling promoting agent according to claim 5, in which the catechin-type tannin is selected from catechins consisting of tea catechin, catechin (C), epicatechin (EC), epicatechin gallate (ECG), epigallocatechin (EGC), gallocatechin gallate (GCG), and epigallocatechin gallate (EGCG).

9. A nonfreezing liquid, which is obtained by dissolving the supercooling promoting agent according to any one of claims 1 to 8 in water or an aqueous solution containing an additive appropriate for an application, in which a content of the supercooling promoting agent in the nonfreezing liquid is 0.01 to 30 g/L.

10. The nonfreezing liquid according to claim 9, in which the additive is a component of a medium for culture of animal or plant cells or a component of a solution for storage of a biological material.

11. The nonfreezing liquid according to claim 9, further including a freezing damage inhibitor at 1 to 40 vol%.

12. The non freezing liquid according to claim11, inwhich the freezing damage inhibitor is one kind or two or more kinds selected from the group consisting of methanol, ethanol, acetamide, dimethyl sulfoxide (DMSO), formaldehyde, ethylene glycol, propylene glycol, glycerin, proline, glucose, sorbitol, sucrose, trehalose, polyethylene glycol, dextran 10-150, polyvinylpyrrolidone (PVP), albumin, Ficoll, and hydroxyethyl starch (HES).

13. The nonfreezing liquid according to claim 9 or 11, in which the nonfreezing liquid further includes a biological material and is cooled to 0 to -15°C.

14. A vitrification solution including a freezing damage inhibitor singly or in combination at 20 to 90 vol% and, as a balance, water or an aqueous solution containing an additive appropriate for an application, in which the vitrification solution further includes the supercooling promoting agent according to any one of claims 1 to 8 at 0.01 to 30 g/L.

15. The vitrification solution according to claim 14, in which a content of the water or the aqueous solution containing an additive appropriate for an application is 40 to 80 vol%.

16. The vitrification solution according to claim 14 or 15, in which the vitrification solution further includes a biological material and is cooled to liquid nitrogen temperature.
